# EUROPEAN PATENT APPLICATION

(11) **EP 4 732 760 A1**
(43) Date of publication of application: **29.04.2026**
(21) Application number: 24858098.7
(22) Date of filing: 15.07.2024
(51) Int. Cl.: A61B 5/00

(54) **SEPARABLE SKIN PARAMETER MEASUREMENT MODULE**

(30) Priority: 31.08.2023 CN 202311117875; 31.08.2023 CN 202322367881 U
(71) Applicant: Beijing Tashan Technology Co., Ltd., Beijing 102308 (CN)
(72) Inventor: SUN, Tengchen, Beijing 102308 (CN); ZHANG, Dahua, Beijing 102308 (CN); WANG, Zhen, Beijing 102308 (CN); ZENG, Fanyou, Beijing 102308 (CN)
(74) Representative: Yang, Shu
(86) International application number: PCT/CN2024/105412
(87) International publication number: WO 2025/044554

(57) **Abstract**

This invention relates to a split-type skin parameter detection module, comprising a handheld wireless detection module and a base, the base detachably supporting the detection module. The detection module includes a first measuring electrode, a second measuring electrode, a CDC (Center for Damping and Controlling the Surface), a memory, a first interface, and an energy storage module. The measuring electrodes are used to contact the skin through an insulating layer to obtain capacitance data reflecting the health status of human skin. The base has a second interface, a controller, and a base processing module, which processes the capacitance data from the memory. This invention utilizes capacitive methods for skin parameter detection to achieve advantages such as ease of placement, structural cost, and direct measurement. Simultaneously, it reduces or even eliminates the generation of the distributed capacitance Cw of the human body to ground, thereby further improving measurement accuracy.

## Description

### Technical Field

This invention relates to the field of skin testing, and in particular to a split-type skin parameter testing module.

### Background Technology

Human skin health status, such as the detection of skin parameters such as moisture content, oil content, and elasticity, is of great significance to dermatology and cosmetology.

Capacitance measurement methods are increasingly favored in skin testing due to their advantages in electrode placement, structural cost, and ability to directly measure skin parameters, avoiding errors from indirect detection. For example, CK's corneal measuring instrument uses the mutual capacitance electric field formed by two measuring electrodes to penetrate human skin. The addition of water causes a change in the dielectric constant of the sensing area. By detecting the mutual capacitance value of the two measuring electrodes, the water content in the skin can be reflected. This measurement method can also be applied to oil measurement.

For methods using capacitance to measure skin parameters, we found that the size of the circuitry in the detection module (such as the probe) and the power supply wires negatively impact detection accuracy. For example, in a scenario where human A holds the detection module to detect human B, assuming A and B are separated but not adjacent, because the power supply wire needs to be connected to ground, and the detection module needs to be held by the person, A will form a loop through the electrode-power supply line-ground-A. Since the circuitry is built into the detection module, its large size causes coupling between B and the circuitry when B is held, creating another loop through the circuit-power supply line-ground-B. This results in two distributed capacitances C <sub> w </sub> between the human body and ground, one for A and one for B. Patent 2023104839645 describes the adverse effects of this distributed capacitance C<sub> w </sub> on skin detection, as its presence reduces detection accuracy. Although we proposed in 2023104839645 that the adverse effects of C<sub> w</sub> can be improved / eliminated by measuring the series capacitance Ca between the electrode and the skin, a combination of methods to reduce or even eliminate C<sub> w</sub> would produce superior accuracy. The above describes the negative impacts of circuit size and power supply wires when A measures B. In the scenario where A holds the detection module to measure himself, for example, when A presses the probe onto his facial skin for detection, the hand and face will form a loop, and Cw will also be generated. In the scenario where A holds the detection module to measure B but the two are in physical contact, Cw will also be generated, which will be detrimental to the accuracy of the detection.

### Summary of the Invention

The purpose of this invention is to utilize capacitive methods for skin parameter detection to achieve advantages such as layout flexibility, structural cost, and direct measurement. At the same time, it reduces or even eliminates the generation of the human body's distributed capacitance Cw to the ground, thereby further improving measurement accuracy.

To address this, a split-type skin component detection module is proposed, comprising:
A handheld wireless detection module is configured to have a first measuring electrode, a second measuring electrode, a capacitance-to-digital converter (CDC), a memory, a first interface, and an energy storage module. The measuring electrode is used to contact the skin through an insulating layer to obtain capacitance data reflecting the health status of human skin. The capacitance-to-digital converter couples each measuring electrode and obtains the capacitance data, which is then transmitted to the memory. The memory is coupled to the first interface. The energy storage module is used to provide the handheld wireless detection module with the electrical energy required for capacitance detection.

The base is configured to detachably support the handheld wireless detection module and has a second interface, a controller, and a base processing module. The second interface is used to interface with the first interface when the handheld wireless detection module is supported on the base. The controller is coupled to the base processing module and the second interface respectively. The base processing module is used to process capacitance data from the memory. The processing is configured to include, but is not limited to, external communication and/or data display.

In the above skin detection solution, the handheld detection module is briefly powered by a built-in energy storage module, which can meet the short-term low power supply requirements of detection. At the same time, the connection between the detection module and the ground is eliminated. Furthermore, by distributing display and/or communication circuits to the base, the circuitry in the detection module is streamlined, and the size of the circuitry, including the circuit board, can be reduced. This enhances the impedance between the human handheld device and the internal circuitry, reduces or even disconnects the coupling between the human handheld device and the internal circuitry. In the scenario where human A holds the detection module to detect human B and A and B are in contact, the connection to the ground is disconnected, and no loops can be formed between A and ground, B and ground, or between A and B. Similarly, in the scenario where A holds the detection module to detect himself, or when A holds the detection module to detect B but the two are in physical contact, the generation of loops is reduced or prevented. Ultimately, the distributed capacitance Cw between the human body and the ground is reduced or eliminated, thereby improving accuracy.

After the handheld detection module performs the detection, the data is temporarily stored in a memory, which can be a power-off data retention type, such as flash. After the detection module finishes detection and is placed back on the base, the data is processed by the controller and the base processing module via an interface.

In the above scheme, the capacitance data reflecting the health status of human skin can include capacitance data such as moisture, oil, and / or elasticity. For versatility, the moisture detection module, oil detection module, and elasticity detection module can all share the same base.
handheld wireless detection module can be configured to retain only the capacitance detection function, achieving optimal decoupling.

As an improvement, the handheld wireless detection module has a grip on the sidewall of its housing to guide the user's hand position. The minimum distance between the grip and the measuring electrodes is configured to be at least 40mm. The grip can provide guidance through features such as shape and/or color, ensuring a safe distance between the user's fingers and the electrodes, thus isolating coupling between them. The minimum distance of at least 40mm is designed to account for the influence of electrode power, area, and human electric field on coupling when detecting relevant skin parameters.

Building upon this, as a further improvement to the proposed solution, the capacitor-to-digital converter (CDC) circuit is arranged on the first PCB board. Considering layout considerations, the first PCB board is implemented using an FPC (Flexible Printed Circuit). The FPC is positioned adjacent to the measuring electrodes, where "adjacent" should be understood as ensuring that the length of the connection line between the measuring electrodes and the CDC is less than 20mm. Analog signals are transmitted on this line segment from the measuring electrodes to the CDC. By controlling the length of this line segment through the adjacent arrangement of the FPC and the measuring electrodes, parasitic capacitance can be effectively controlled, further improving accuracy. More preferably, a second PCB board is also provided, using a rigid board for support. The memory and /or energy storage module is arranged on the second PCB board (in a scheme where the controller is placed within the detection module, the controller and /or energy storage module are arranged on this board). After the first and second PCB boards are separated, they are coupled via transmission lines. Digital signals are transmitted on this transmission line from the first PCB board to the second PCB board, which does not affect parasitic capacitance. Therefore, the arrangement and position of this transmission line segment can have greater freedom and fewer restrictions. As for the arrangement of the transmission line between the second PCB board and the first interface, it can be arranged along the inner wall of the detection module housing. To avoid coupling with the human hand, a suspended central structure can be built into the housing of the detection module. The central structure is kept away from the side wall due to suspension. This section of the transmission line is arranged in the central structure to ensure that it is kept away from the human hand on the gripping part.

In this invention, to fully isolate the coupling between the fingers or palm and the second PCB board, the shortest distance between the second PCB board and the grip portion is configured to be at least 1 mm. Furthermore, it is preferable that the second PCB board and the grip portion are offset in the horizontal direction. This ensures that their horizontal projections do not intersect, reducing the coupling area between the grip and the PCB, thus minimizing the impact of parasitic capacitance and ensuring accuracy. Preferably, the measuring electrodes and /or the first interface are distributed along the Z-axis of the handheld wireless detection module; the grip portion is distributed along the X-axis of the handheld wireless detection module and offset from the second PCB board along the Z-axis, forming a relatively perpendicular distribution to minimize coupling effects.

As a further improvement to this design, the grip is equipped with a switch. This switch, which requires pressing, enhances the guidance of the hand position. During use, the switch can trigger the detection action, such as when the electrodes of the detection module are pressed against the skin through the insulating layer, allowing the user to manually control the detection. In this invention, since a power supply module is used instead of a wired power supply, the stored power cannot be set too large (proportional to its volume) to avoid enhancing coupling. Therefore, automatic /all-weather detection is unsuitable. The user manually controls the power supply briefly when ready, which is advantageous for achieving the function while ensuring proper coupling.

As an alternative improvement, the energy storage module can be either a battery or a capacitor. Considering that batteries are more prone to failure than capacitors, and given the relatively low power demand of the detection module under the above design, a capacitor is preferred for powering the detection module. Furthermore, the base is equipped with a charging circuit to charge the energy storage module when the handheld detection module is placed on the base.

As an alternative improvement, the base processing module is provided with a wireless communication module and/or a wired interface for external communication and coupled to the second interface; and/or the base processing module is provided with a display module coupled to the second interface.

As an alternative improvement, the detection module and the base are configured to be magnetically fixed to each other, allowing for convenient fixing and separation.

As another improvement, the ratio of the series capacitance Ca1 between the first measuring electrode and the human body and the series capacitance Ca2 between the second measuring electrode and the human body is configured using a known scaling factor k. The scaling factor k can be set to 1, or it can be different. A scheme where k equals 1 can be achieved by setting the normal projection areas of the first and second measuring electrodes corresponding to the human body to be the same, and the distance between the first and second measuring electrodes and the human body to be the same. This scheme is more conducive to reducing computational power and improving detection speed, and is considered a preferred solution. For schemes where k is not equal to 1, it can be achieved by setting different areas and/or distances, for example, setting the area of the first measuring electrode to half the area of the second measuring electrode, or setting the distance between the first and second measuring electrodes to 1/3 of the distance between the second measuring electrode and the human body, etc.

Furthermore, in the CDC (Chemical Density Detector), based on the contact between the first and second measuring electrodes and the skin via an insulating layer, and the known scaling factor k, a scheme for calculating skin component information using series capacitance Ca can be combined with the above scheme to achieve higher performance accuracy and reliability. Specifically, a capacitance-to-digital conversion circuit can be configured to couple each measuring electrode and acquire the first and second capacitances. The first capacitance is configured as one of the following: a first self-capacitance measurement value acquired through the first measuring electrode, a second self-capacitance measurement value acquired through the second measuring electrode, a third self-capacitance measurement value acquired through parallel connection of the first and second measuring electrodes, or a first mutual capacitance measurement value acquired through the first and second measuring electrodes. The second capacitance is configured as one of the other three. The processing module is used to construct a first equation based on the first capacitance, with the human body-to-ground distributed capacitance Cw and the corresponding series capacitance as variables, and a second equation based on the second capacitance, with the human body-to-ground distributed capacitance Cw and the corresponding series capacitance as variables. The system of equations composed of the first and second equations and the scaling factor k is used to calculate the series capacitance Ca1 or Ca2 to output the skin component information. In practical scenarios, during testing, the first and second measuring electrodes are tightly attached to the skin through an insulating layer, with a fixed distance between the electrodes and the skin. Thus, by setting the area ratio of the first and second measuring electrodes after manufacturing, the proportionality coefficient k can be determined through structural settings (the series capacitance of one electrode is Ca, and the series capacitance of the other is k*Ca). Based on this, the self-capacitance or mutual capacitance of the two measuring electrodes is used. Since both self-capacitance and mutual capacitance are composed of Cw and Ca, each measurement can construct a functional equation of Cw and Ca. By eliminating Cw using the system of equations formed by the two equations, a monotonic function of Ca and the first and second capacitances can be constructed. Then, Ca can be solved using the first and second capacitances obtained from the two measurements. Because Cw is eliminated, the measurement error caused by Cw can be eliminated, thus accurately measuring and calculating Ca to reflect the skin component content, such as moisture content and oil content. Furthermore, by using capacitance-to-digital converter (CDC) circuits, such as ADI7142 and ADI7147, and employing Δ-Σ modulation, the measured capacitance value is directly converted into a digital value by repeatedly charging and discharging the measured capacitor and comparing it with a reference capacitor (see: US Patent Number: 5,134,401). This can improve the measurement sensitivity of capacitance to the 1ff level, easily meeting the capacitance measurement sensitivity requirements of measurement systems. In particular, these chips are designed with multiple channels, making circuit design simple and convenient, thereby effectively reducing costs and installation difficulty.

In the above solution, the controller is placed on the base, meaning all processing logic is handled on the base. In a secondary solution, the controller can be configured on the detection module. Therefore, another separate skin component detection module is provided, including:
handheld wireless detection module is configured to have a first measuring electrode, a second measuring electrode, a capacitance-to-digital conversion circuit, a controller, a first interface, and an energy storage module. The measuring electrode is used to contact the skin through an insulating layer to obtain capacitance data reflecting the health status of human skin. The capacitance-to-digital conversion circuit couples each measuring electrode and transmits the obtained capacitance data to the controller. The controller is coupled to the first interface. The energy storage module is used to provide the handheld wireless detection module with the electrical energy required for capacitance detection.

The base is configured to detachably support the handheld wireless detection module and has a second interface and a base processing module. The second interface is used to interface with the first interface when the handheld wireless detection module is supported on the base. The base processing module is coupled to the second interface and is used to process capacitance data from the controller. The processing is configured to include, but is not limited to, external communication and /or data display.

In this scheme, other improvements, such as the distance configuration between the grip and the measuring electrode , the structural configuration of the first and second PCBs, the central structural component, and the transmission line, the distribution of the electrodes, grip, and interface, and the coefficient settings between the electrodes, can all be referenced from the scheme of placing the controller on the base, and will not be elaborated here.

### Brief Description of Figures

Figure 1 shows a schematic diagram of the split-type skin component detection module;
Figure 2 shows a schematic diagram of the handheld wireless detection module;
Figure 3 shows a schematic diagram of the detection electrode assembly structure;
Figure 4 shows a schematic diagram of the transmission line assembly and the second PCB structure;
Figure 5 shows a schematic diagram of the distribution of the hand gripping parts;
Figure 6 shows a schematic diagram of the base structure;
Figure 7 shows a schematic diagram of the detection electrode distribution structure;
Figure 8 shows a schematic diagram of the human body-to-ground distributed capacitance generated by the first and second detection electrodes.

### Detailed Description

The technical solutions of the present invention will be clearly and completely described below with reference to the accompanying drawings in the embodiments of the present invention.

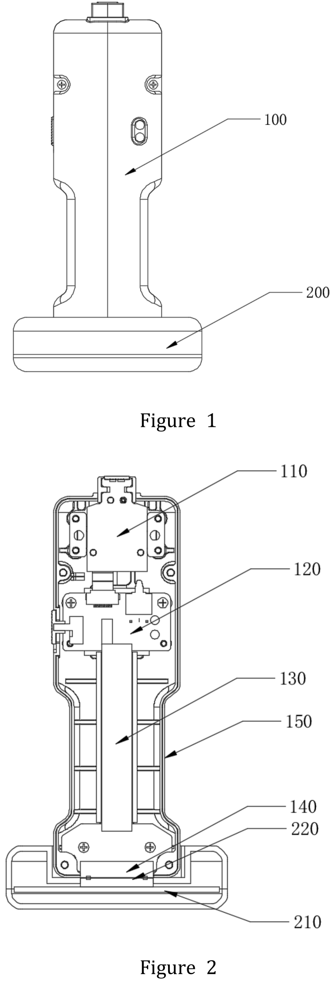

As shown in Figure 1, the split-type skin component detection module mainly consists of two parts: a handheld wireless detection module 100 and a base 200. The base 200 detachably supports the handheld wireless detection module 100 and transmits data through a fixed interface.

As shown in Figure 2, the handheld wireless detection module 100 mainly comprises a detection electrode assembly 110, a second PCB 120, a transmission line assembly 130, a first interface 140, and a hand-held gripping area 150. The detection electrode assembly 110 includes detection electrodes that contact the skin of the person being tested through an insulating layer to acquire skin parameter information. After signal processing, the signal is transmitted to the second PCB 120 via the detection electrode transmission line 114. The second PCB 120 includes an energy storage module and a memory.

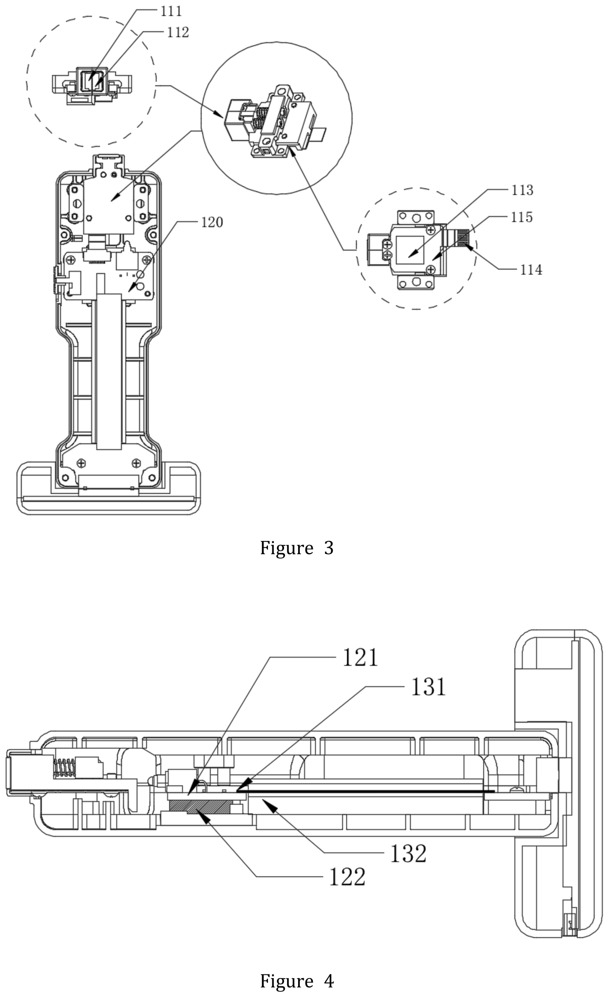

As shown in Figure 3, the detection electrode assembly 110 mainly includes a first detection electrode 111, a second detection electrode 112, a capacitance information processing module 113, a detection electrode transmission line 114, and a first PCB 115. The first detection electrode 111 and the second detection electrode 112 are in contact with human skin via an insulating layer, which includes, but is not limited to, an insulating film or an insulating coating. The capacitance information processing module 113 mainly includes a capacitance-to-digital conversion circuit to obtain the capacitance change information of the first detection electrode 111 and the second detection electrode 112, thereby deriving the skin characteristic parameters of the test subject. The first PCB 115 is configured as an FPC. The capacitance information processing module 113 is arranged close to the first detection electrode 111 and the second detection electrode 112 to reduce interference. The capacitance change information of the first detection electrode 111 and the second detection electrode 112 is processed by the capacitance information processing module 113, converted into a digital signal, and transmitted to the second PCB 120 via the detection electrode transmission line.

As shown in Figure 4, the second PCB 120 mainly includes an adapter PCB 121, an energy storage module 122, and a transmission line assembly 130 mainly includes a data transmission line 131 and a central suspension structure 132. The second PCB 120 includes the energy storage module 122, which can be configured with small batteries or capacitors, such as farad capacitors, to provide short-term testing energy. The central suspension structure 132 of the transmission line assembly 130 is fixed to the outer casing, and the data transmission line 131 is placed inside the central suspension structure 132, away from the hand grip area 150, reducing the influence of the human body on the measurement results. When human body A holds the handheld wireless detection module 100 to detect human body B, the power supply wire needs to be connected to the ground. Since A holds the handheld wireless detection module 100, human body A forms a loop through the electrode-power supply line-ground-A, while B forms another loop through the electrode-power supply line-ground-B. This results in A and B each forming a distributed capacitance Cw to ground. By using a capacitor to supply power and misaligning the hand holding part 150 with the internal PCB to disconnect it from the ground, loops cannot be formed between A and ground, B and ground, or between A and B. Similarly, in scenarios where A holds the detection module to detect himself or when A holds the wireless detection module to detect B but the two are in physical contact, the generation of loops is reduced or prevented, reducing or even eliminating the distributed capacitance Cw to ground, thus improving accuracy.

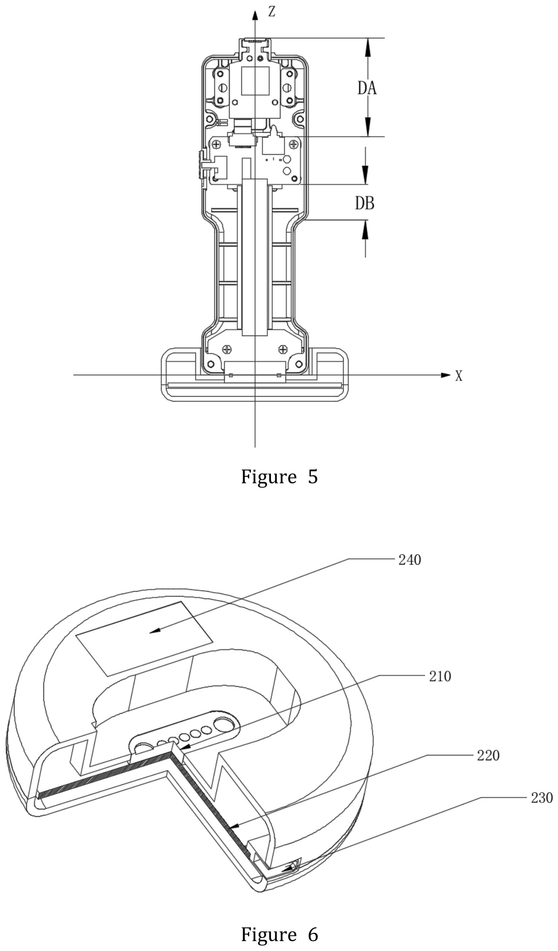

As shown in Figure 5, the hand gripping part 150 and the transmission line assembly 130 do not contact each other in the X direction and a gap needs to be maintained. The shortest distance between the detection electrode of the detection electrode assembly 110 and the hand gripping part 150 is at least 40 mm, as shown by distance DA in Figure 5. The distance between the second PCB 120 and the hand gripping part 150 is at least 1 mm, as shown by distance DB in Figure 5.

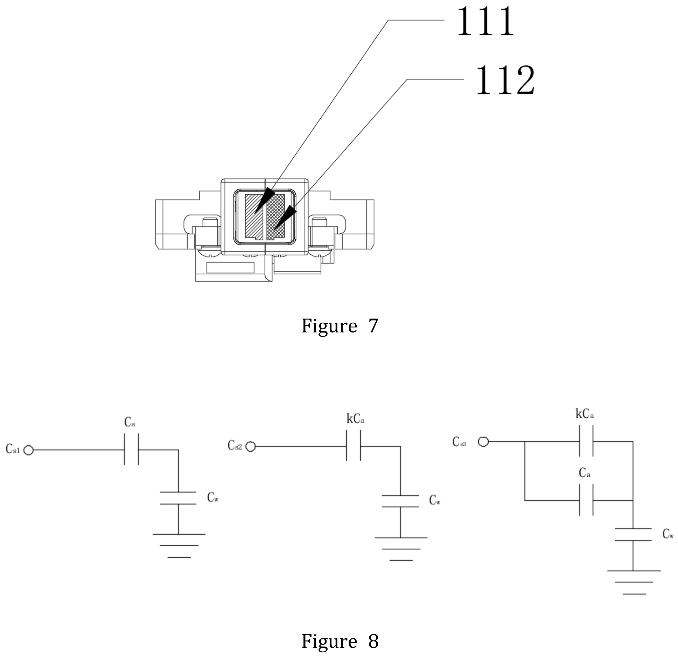

As shown in Figure 6, the base 200 mainly includes a second interface 210, a base processing module 220, an external interface 230, and a display device 240. The second interface 210 interfaces with the first interface 140 of the handheld wireless detection module 100, and is then connected to the second PCB via a transmission line assembly 130, enabling data transmission between the handheld wireless detection module 100 and the base 200. Simultaneously, it can charge the energy storage module 122 to meet short-term measurement needs. The second interface 210 and the first interface 140 can be configured to magnetically attract each other, relying on mechanical fixation and mutual magnetic force to ensure contact and avoid signal interference caused by poor contact. The base processing module 220 includes a communication module, which can transmit data information to terminal devices or servers. It can also achieve wired data transmission through the external interface 230. The display device 240 can display detection results and other information to the operator in real time, providing operation progress and prompts. The base processing module 200 of the base 200 transfers some functions to the base 200, slimming down the handheld wireless detection module 100 and achieving a smaller size for easier use.
, the first detection electrode 111 and the second detection electrode 112 form series capacitances Ca1 and Ca2 with the human body, respectively. The ratio of Ca1 to Ca2 is configured as K, where the proportionality coefficient K can be configured as 1 or not equal to 1. The value of the proportionality coefficient K depends on the change in the projected area of the detection electrode and the human body, and the change in projected area is selected during the design process of the detection electrode. As shown in Figure 8, the ratio of Ca1 to Ca2 is configured as K, that is, the series capacitance of one electrode is Ca, and the other is KCa. During the test, the self-capacitance measurement value Cs1 of the first detection electrode 111, the self-capacitance measurement value Cs2 of the second detection electrode 112, and the third self-capacitance measurement value Cs3 of the first detection electrode 111 and the second detection electrode 112 in parallel are obtained. Two of these measurement values are randomly selected to establish an equation system, and the elimination operation Cw is performed to eliminate the influence of the distributed capacitance of the human body to the ground, thereby accurately measuring the skin feature information value of the test subject.

Finally, it should be noted that the above embodiments are only used to illustrate the technical solutions of the present invention, and are not intended to limit the scope of protection of the present invention. Although the present invention has been described in detail with reference to preferred embodiments, those skilled in the art should understand that modifications or equivalent substitutions can be made to the technical solutions of the present invention without departing from the essence and scope of the technical solutions of the present invention.

## Claims

1. A split-type skin parameter detection module, **characterized in that** it includes:
a handheld wireless detection module (100) is configured to have a first measuring electrode (111), a second measuring electrode (112), a capacitance-to-digital conversion circuit, a memory, a first interface (140), and an energy storage module (122). The measuring electrode (111, 112) is used to contact the skin through an insulating layer to obtain capacitance data reflecting the health status of human skin. The capacitance-to-digital conversion circuit couples each measuring electrode (111, 112) and obtains the capacitance data, which is then transmitted to the memory. The memory is coupled to the first interface (140). The energy storage module (122) is used to provide the handheld wireless detection module (100) with the electrical energy required for capacitance detection.
a base (200) is configured to detachably support the handheld wireless detection module (100) and has a second interface (210), a controller, and a base processing module (220), the second interface (210) is used to interface with the first interface (140) when the handheld wireless detection module (100) is supported on the base (200), the controller is coupled to the base processing module (220) and the second interface (210) respectively, the base processing module (220) is used to process capacitance data from the memory, the processing is configured to include, but is not limited to, external communication and /or data display.

2. The split-type skin parameter detection module according to claim 1 is **characterized in that**:
the handheld wireless detection module (100) has a grip (150) on the sidewall of its housing to guide the user's hand position, and the shortest distance between the grip (150) and the measuring electrode (111, 112) is configured to be at least 40 mm.

3. According to claim 2, the split-type skin parameter detection module is **characterized in that**: the capacitor-to-digital conversion circuit is arranged on the first PCB board (115), and the first PCB board (115) is arranged adjacent to the measuring electrode (111, 112).

4. The split-type skin parameter detection module according to claim 3 is **characterized in that**:
the memory and /or energy storage module (122) is arranged on the second PCB board (120), and the first PCB board (115) is separated from the second PCB board (120) and coupled by a transmission line (130).

5. According to claim 4, the split-type skin parameter detection module is **characterized in that**: the outer shell of the handheld wireless detection module (100) has a suspended central structural component (132), and the transmission line (131) between the second PCB board (120) and the first interface (140) is arranged in the central structural component (132) so as to be away from the gripping part (150).

6. According to claim 4, the split-type skin parameter detection module is **characterized in that**: the shortest distance between the second PCB board (120) and the gripping part (150) is configured to be at least 1 mm.

7. According to claim 6, the split-type skin parameter detection module is **characterized in that**: the second PCB board (120) and the gripping part (150) are misaligned in the horizontal direction.

8. The split-type skin parameter detection module according to claim 7 is **characterized in that**:
measuring electrodes (111, 112) and /or the first interface (140) are distributed along the Z-axis of the handheld wireless detection module (100);
The gripping part (150) is located in the X-axis direction of the handheld wireless detection module (100) and is offset from the second PCB board (120) in the Z-axis direction.

9. The split-type skin parameter detection module according to claim 2 is **characterized in that**: the grip part (150) is provided with a switch.

10. The split-type skin parameter detection module according to claim 1 is **characterized in that**: the energy storage module (122) is configured as a capacitor.

11. The split-type skin parameter detection module according to claim 1 is **characterized in that**:
Ca1 between the first measuring electrode (111) and the human body and the series capacitance Ca2 between the second measuring electrode (112) and the human body is configured to a known proportionality coefficient k.

12. A split-type skin parameter detection module, **characterized in that** it includes:
a handheld wireless detection module (100) is configured to have a first measuring electrode (111), a second measuring electrode (112), a capacitance-to-digital conversion circuit, a controller, a first interface (140), and an energy storage module (122). The measuring electrode (111, 112) is used to contact the skin through an insulating layer to obtain capacitance data reflecting the health status of human skin. The capacitance-to-digital conversion circuit couples each measuring electrode (111, 112) and transmits the obtained capacitance data to the controller. The controller is coupled to the first interface (140). The energy storage module (122) is used to provide the handheld wireless detection module (100) with the electrical energy required for capacitance detection.
a base (200) is configured to detachably support the handheld wireless detection module (100) and has a second interface (210) and a base processing module (220). The second interface (210) is used to interface with the first interface (140) when the handheld wireless detection module (100) is supported on the base (200). The base processing module (220) is coupled to the second interface (210) and is used to process capacitance data from the controller. The processing is configured to include, but is not limited to, external communication and /or data display.
